(19) Europäisches Patentamt
European Patent Office
Office européen des brevets

(11) Publication number: 0 378 791
A2

# (12) EUROPEAN PATENT APPLICATION

(21) Application number: 89122266.3

(22) Date of filing: 02.12.89

(51) Int. Cl.[5]: C07D 417/04, C07D 403/04, C07D 413/04, C07D 401/04, A01N 43/82, A01N 43/56, A01N 43/76, A01N 43/80, A01N 43/78, A01N 43/40, A01N 43/54

(30) Priority: 15.12.88 JP 314859/88

(43) Date of publication of application: 25.07.90 Bulletin 90/30

(84) Designated Contracting States: BE CH DE FR GB IT LI NL

(71) Applicant: NIHON TOKUSHU NOYAKU SEIZO K.K.
Itohpia Nihonbashi Honcho Building 7-1, Nihonbashi Honcho 2-chome
Chuo-ku Tokyo 103(JP)

(72) Inventor: Kume, Toyohiko
6-7-8, Asahigaoka
Hino-shi Tokyo(JP)
Inventor: Goto, Toshio
3454-21, Honmachida
Machida-shi Tokyo(JP)
Inventor: Kamochi, Atsumi
2-24-10, Higashi-Toyoda
Hino-shi Tokyo(JP)
Inventor: Yanagi, Akihiko
2-1200-1, Nagabuchi
Oume-shi Tokyo(JP)
Inventor: Miyauchi, Hiroshi
39-15, Namiki-cho
Hachioji-shi Tokyo(JP)

(74) Representative: Schumacher, Günter, Dr. et al
c/o Bayer AG Konzernverwaltung RP
Patentabteilung
D-5090 Leverkusen 1 Bayerwerk(DE)

## (54) 3,4-Dimethyl-pyrrolidine derivatives.

(57) Novel 3,4-dimethyl-pyrrolidine derivatives of the formula (I)

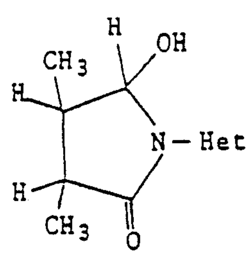

and the use of the new compounds as selective herbicides.

EP 0 378 791 A2

## 3,4-DIMETHYL-PYRROLIDINE DERIVATIVES

The present invention relates to novel 3,4-dimethyl-pyrrolidine derivatives, to a process for their preparation, and to their use as herbicides.

It has already been disclosed that certain 3,4-dimethyl-2-hydroxy-5-oxo-2,5-dihydropyrrole derivatives are usefull as pesticides for plants and animals (see Japanese Laid-Open Patent application No.23965/1978).

There have now been found novel 3,4-dimethyl-pyrrolidine derivatives of the formula (I).

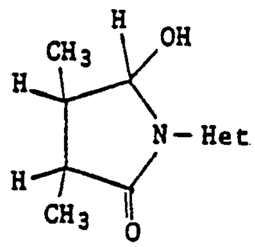

(I)

wherein Het represents a 5- or 6-membered heterocyclic radical containing 1 to 4 heteroatoms and optionally having a fused benzene ring, with the proviso that at least one of the heteroatoms is nitrogen, and that, when there are at least two heteroatoms, these heteroatoms may be identical or different from each other, and wherein Het may optionally be mono-, di-, tri- or tetra-substituted with identical or different radicals selected from the class consisting of halogen, $C_{1-8}$ alkyl, $C_{1-8}$ haloalkyl, unsubstituted or methyl-substituted $C_{3-6}$ cycloalkyl, $C_{1-4}$ alkoxy, $C_{1-4}$ haloalkoxy, $C_{1-4}$ alkylsulfonyl and $C_{1-4}$ haloalkylsulfonyl.

3,4-dimethyl-pyrrolidine derivatives of the formula (I) are obtained

when

3,4-dimethyl-2,5-dioxopyrrolidine derivatives of the formula (II)

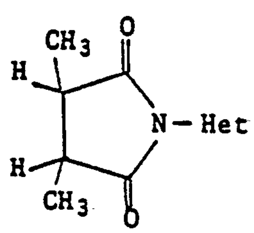

(II)

wherein Het has the meaning stated above,

are reacted with sodium borohydride, in the presence of inert solvents.

The 3,4-dimethyl-pyrrolidine derivatives exhibit powerful herbicidal properties.

Surprisingly, the 3,4-dimethyl-pyrrolidine derivatives according to the invention exhibit a substantially greater herbicidal action than those known from the prior art, for instance, aforementioned Japanese Laid-Open Patent application No.23965/1978.

Among the 3,4-dimethyl-pyrrolidine derivatives according to the invention, of the formula (I), preferred compounds are those wherein

Het represents a 5- or 6-membered heterocyclic radical containing 1 to 3 heteroatoms and optionally having a fused benzene ring, with the proviso that at least one of the heteroatoms is nitrogen, and that, when there are at least two heteroatoms, these heteroatoms may be identical or different from each other, and wherein Het may optionally be mono-, di- or tri-substituted with identical or different radicals selected from the class consisting of fluorine, chlorine, $C_{1-6}$ alkyl, $C_{1-6}$ haloalkyl, 1-methyl-cyclopropan-1-yl, 1-methyl-cyclopentan-1-yl, 1-methyl-cyclohexan-1-yl, methoxy, ethoxy, trifluoromethoxy, 2,2,2-trifluoroethoxy, methylsulfonyl, ethylsulfonyl, n-propylsulfonyl, iso-propylsulfonyl, tert-butylsulfonyl, trifluoromethylsulfonyl and 2,2,2-trifluoroethylsulfonyl.

Very particularly prefered 3,4-dimethyl-pyrrolidine derivatives of the formula (I) are those wherein Het represents a 5- or 6-membered heterocyclic radical containing 1 to 3 heteroatoms and optionally having a fused benzene ring, with the proviso that at least one of the heteroatoms is nitrogen, and that, when there are at least two heteroatoms, these heteroatoms may be identical or different from each other, and wherein

Het may optionally be mono- or di-substituted with identical or different radicals selected from the class consisting of fluorine, chlorine, methyl, iso-propyl, tert-butyl, 1-ethyl- 1-methylpropyl, 1,1-dimethylpropyl, 2-chloro-1,1-dimethylethyl, 2-fluoro-1,1-dimethylethyl, trifluoromethyl, 1-methylcyclopropan-1-yl, trifluoromethoxy, methylsulfonyl, ethylsulfonyl, iso-propylsulfonyl, tert-butylsulfonyl and trifluoromethylsulfonyl.

Examples of the heterocyclic radicals optionally having a fused benzene ring, represented by Het in the general formula (I) of the present compounds, include those selected from the class consisting of pyrrole, furan, thiophene, pyrazole, imidazole, oxazole, isoxazole, thiazole, isothiazole, triazole, oxadiazole, thiadiazole pyridine, pyrimidine, pyridazine, pyrazine, oxazine, thiazine, triazine oxadiazine, thiadiazine, indole, iso-indole, benzofuran, benzothiophene, benzimidazole, indazole, benzoxazole, benzothiazole, quinoline, isoquinoline, cinnoline, quinazoline, quinoxaline, phthalazine, naphthyridine, benzoxazine and benzothiazine.

Preferred examples of the heterocyclic radicals are those selected from the class consisting of pyrazole, imidazole, oxazole, isoxazole, thiazole, isothiazole, triazole, oxadiazole, thiadiazole, pyridine, pyrimidine, triazine, benzimidazole, benzoxazole, benzothiazole, quinoline, quinazoline and quinoxaline.

More preferred examples of the heterocyclic radicals are those selected from the class consisting of pyrazole, oxazole, isoxazole, thiazole, isothiazole, oxadiazole, thiadiazole, pyridine, pyrimidine, benzoxazole, and benzothiazole.

If, for example, N-(5-tert-butyl-1,3,4-thiadiazol-2-yl)-3,4-dimethyl-2,5-dioxopyrrolidine and sodium borohydride are used as starting materials, the course of the reaction can be represented by the following equation:

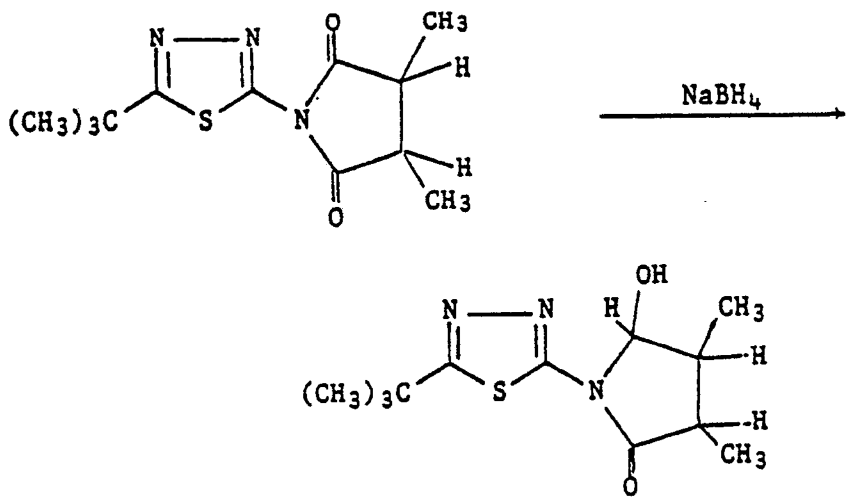


The compounds of the formula (II) mean compounds based on the above definition of Het, preferably compounds based on the above preferred definitions.

The compounds of the formula (II) in part are novel, and can be prepared, for instance, according to a process, wherein compounds of the formula (III)

$H_2N$-Het (III)

wherein Het has the meaning stated above,

are reacted with 2,3-dimethylsuccinic anhydride, if appropriate in the presence of acid, in the presence of inert solvents.

Compounds of the formula (III) are well known in the field of organic chemistry. Examples of the compounds (III) are:

2-amino-5-tert-butyl-1,3,4-thiadiazole;
3-amino-5-tert-butylisoxazole;
5-amino-3-tert-butylisoxazole;
2-amino-5-tert-butyl-1,3,4-oxadiazole;
2-amino-5-tert-butyloxazole;
3-amino-5-tert-butylpyrazole;
2-amino-5-(2-chloro-1,1-dimethylethyl)-1,3,4-thiadiazole;
2-amino-5-(iso-propylsulfonyl)-1,3,4-thiadiazole, and the like.

As appropriate diluents to be used in carrying out the process for the preparation of the formula (I), any kind of inert solvents can be mentioned.

Examples of the diluents are water; aliphatic, cycloaliphatic and aromatic hydrocarbons optionally chlorinated, e.g. hexane, cyclohexane, petroleum ether, ligroin, benzene, toluene, xylene, methylene chloride, chloroform, carbon tetrachloride, 1-2-dichloroethane, chlorobenzene, dichlorobenzene and the like; ethers such as diethyl ether, methyl ethyl ether, di-iso-propyl ether, dibutyl ether, dioxane, tetrahydrofuran, 1,2-dimethoxyethane and the like; such as methanol, ethanol, iso-propanol, butanol, ethylene glycol and the like; acid amides such as dimethylformamide, dimethylacetamide and the like; and bases such as pyridine and the like.

The reaction temperature of the process may vary in a fairly wide range. In general, the reaction is carried out at a temperature of about 0 to 150 °C, preferably a temperature of about 20 to 80 °C. It is preferred to carry out the reaction under the normal pressure, although a higher or lower pressure can also be used.

In conducting the process, for example, about 0.5 to 1.5 moles of sodium borohydride per mole of the compounds of the formula (II) are employed to react them with each other to obtain the aimed compounds of the formula (I).

The active compounds according to the invention can be used as defoliants, desiccants, agents for destroying broad-leaved palnts and, especially, as weedkillers. By weeds, in the broadest sense, there are to be understood all plants which grow in locations where they are undesired. Whether the substances according to the invention act as total or selective herbicides depends essentially on the amount used.

The active compouns according to the invention can be used, for example, in connection with the following plants:

Dicotyledon weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver and Centaurea.

Dicotyledon cultures of the genera: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis and Cucurbita.

Monocotyledon weeds of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus and Apera.

Monocotyledon cultures of the genera: Oryza, Zea, Triticum, Hordeum, Avena, Secale, Sorghum, Panicum, Saccharum, Ananas, Asparagus and Allium.

However, the use of the active compounds according to the invention is in no way restricted to these genera, but also extends in the same manner to other plants.

The compounds are suitable, depending on the concentration, for the total combating of weeds, for example on industrial terrain and rail tracks, and on paths and squares with or without tree plantings. Equally, the compounds can be emplyed for combating weeds in perennial cultures, for example afforestations, decorative tree plantings, orchards, vineyards, citrus groves, nut orchards, banana plantations, coffee plantations, tea plantations, rubber plantations, oil palm plantations, cocoa plantations, soft fruit plantings and hopfields, and for the slective combating of weeds in annual cultures.

The active compounds can be converted into the customary formulations, such as solutions, emulsions, wettable powders, suspensions, powders, foams, pastes, granules, aerosols, natural and synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl napthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethyl-sulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorganic and organic meals, and granules of organic material such as sawdust, coconut shells, maize cobs and tobacco stalks.

As emulsifying and/or foam-forming agents there may be used non-ionic and anionic emulsifiers, such as polyoxyethylene-fatty acid esters, polyoxyethylene-fatty alcohol ethers, for example alkylaryl polyglycol ethers, alkyl sulphonates, alkyl sulphates, aryl sulphonates as well as albumin hydrolysis products. Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyanine dyestuffs, and trace nutrients, such as salts of iron, manganese boron, copper, cobalt, molybdenum and zinc.

The formulations in general contain from 0.1 to 95 per cent by weight of active compound, preferably from 0.5 to 90 per cent by weight.

The active compounds according to the invention, as such or in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellants, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomising or scattering.

The active compounds according to the invention can be applied either before or after emergence of the plants.

They can also be incorporated into the soil before sowing. They are used, in particular, after emergence of the plants.

The amount of active compound used can vary within a substantial range. It depends essentially on the nature of the desired effect. In general, the amounts used are between 0.001 and 5 kg of active compound per hectare of soil surface, preferably between 0.05 and 3 kg per ha.

The preparation and use of the active compounds according to the invention can be seen from the following examples.

Preparative Examples:

Example 1

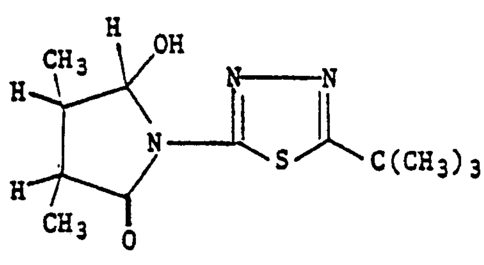

2-Amino-5-tert-butyl-1,3,4-thiadiazole (1.57 g) and 2,3-dimethylsuccinic anhydride (1.41 g) were heated in toluene (30 ml) under reflux for 1 hour, and then acetic acid (5 ml) was added thereto. The reaction

mixture was further heated under reflux for 5 hours.

After the reaction had been completed, the reaction mixture was washed with water and then with a saturated aqueous solution of sodium hydrogen carbonate, and dried over anhydrous magnesium sulfate.

The toluene was distilled off under a reduced pressure, and the residual imide (2.63 g) was dissolved in methanol (50 ml). To the resulting solution was added sodium borohydride (0.19 g). The reaction mixture was stirred for 5 hours. After the reaction subsided, acetic acid (0.5 ml) was added to the reaction mixture, and the solvent was distilled off under a reduced pressure. The residue was admixed with water, and the resulting mixture was extracted with dichloromethane (40ml x 2), and the extract was combined and dried over anhydrous magnesium sulfate. The solvent was distilled off under a reduced pressure, and the resultant residue was washed with a small amount of ether. N-(5-tert-butyl-1,3,4-thiadiazol-2-yl)-3,4-dimethyl-2-hydroxy-5-oxo-pyrrolidine (2.31 g) was obtained. mp. 152 - 153° C.

Table 1 below shows compounds of the invention which may be obtained by the same method as above.

Table 1

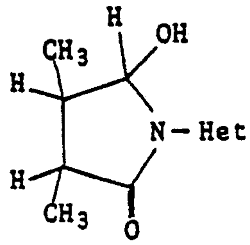

| Compound No. | Het | mp. (°C) |
|---|---|---|
| 1 | 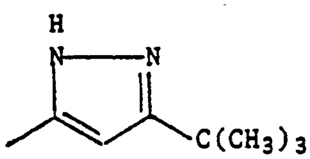 | 205 - 208 |
| 2 | 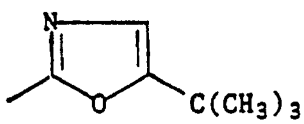 | 114 - 117 |
| 3 | 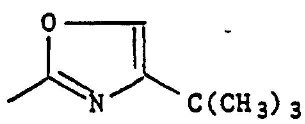 | Viscous oil |
| 4 | 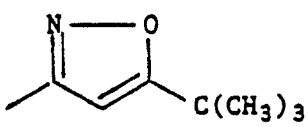 | Viscous oil |
| 5 | 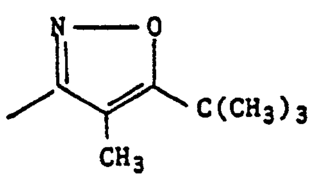 | Viscous oil |
| 6 | 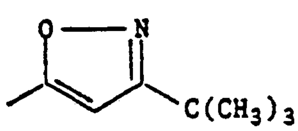 | Viscous oil |

| Compound No. | Het | mp. (°C) |
|---|---|---|
| 7 | 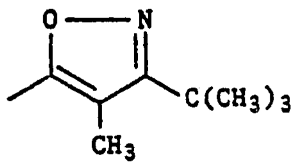 | Viscous oil |
| 8 | 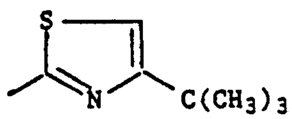 | Viscous oil |
| 9 | 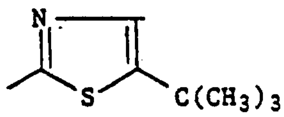 | |
| 10 | 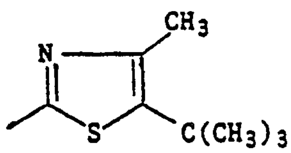 | |
| 11 | 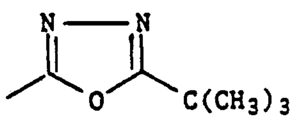 | 175 - 178 |
| 12 | 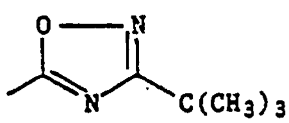 | |
| 13 | 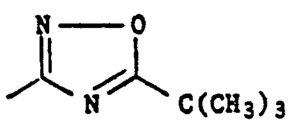 | |
| 14 | 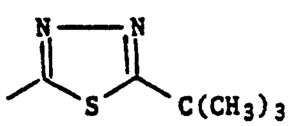 | 152 - 153 |

| Compound No. | Het | mp. (°C) |
|---|---|---|
| 15 | 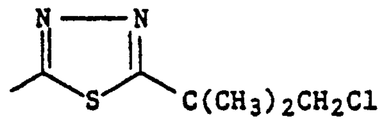 | 115 - 117 |
| 16 | 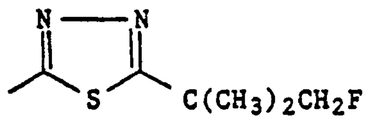 | |
| 17 | 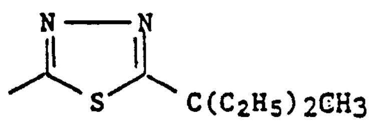 | |
| 18 | 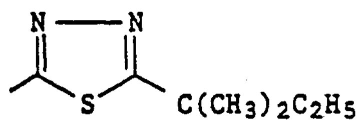 | 214 - 216 |
| 19 | 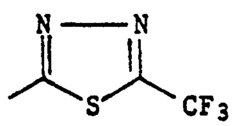 | 108 - 113 |
| 20 | 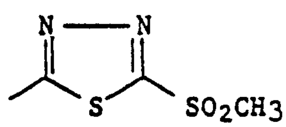 | 173 - 177 |
| 21 | 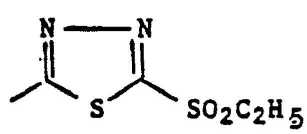 | |
| 22 | 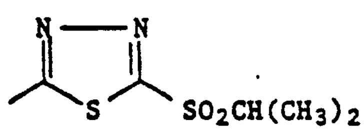 | 203 - 205 |

| Compound No. | Het | mp. (°C) |
|---|---|---|
| 23 | 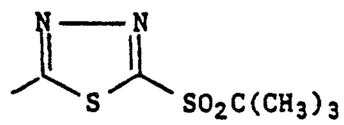 | |
| 24 | 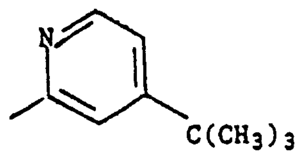 | |
| 25 | 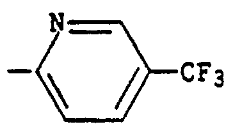 | 93 - 98 |
| 26 | 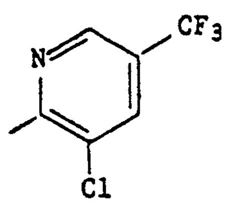 | |
| 27 | 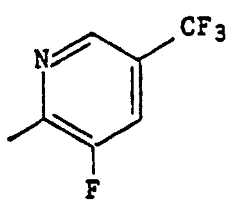 | |
| 28 | 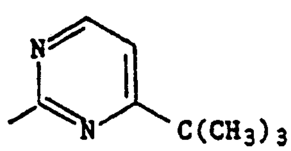 | |
| 29 | 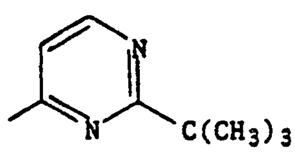 | |

| Compound No. | Het | mp. (°C) |
|---|---|---|
| 30 | 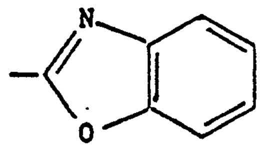 | |
| 31 | 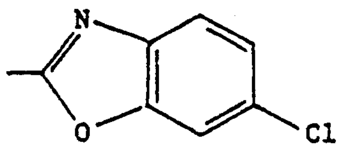 | |
| 32 | 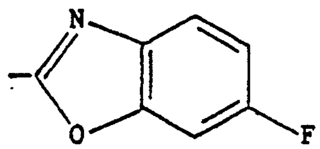 | |
| 33 | 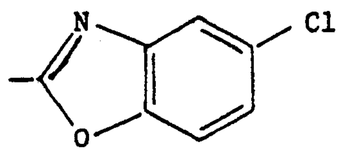 | |
| 34 | 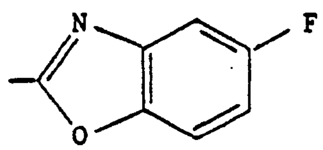 | |
| 35 | 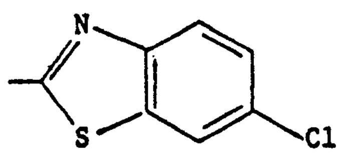 | |
| 36 | 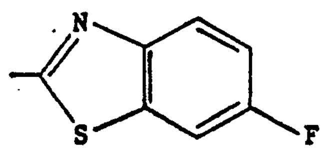 | |

| Compound No. | Het | mp. (°C) |
|---|---|---|
| 37 | 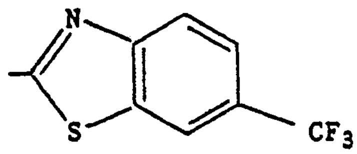 | |
| 38 | 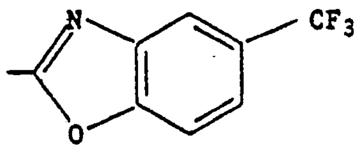 | |
| 39 | 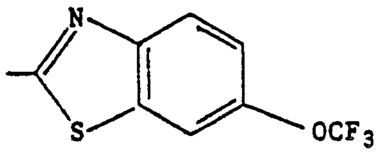 | |
| 40 | 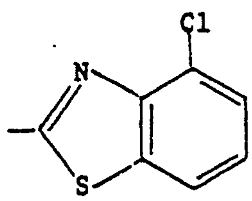 | |
| 41 | 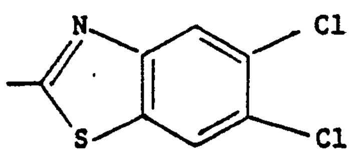 | |

Biotest Examples

Known compounds employed for comparison:

E-1

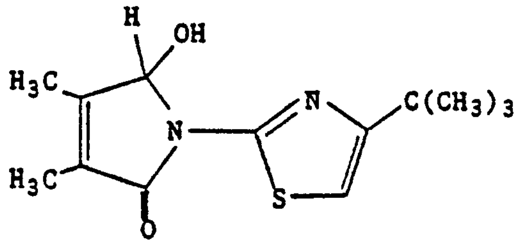

E-2

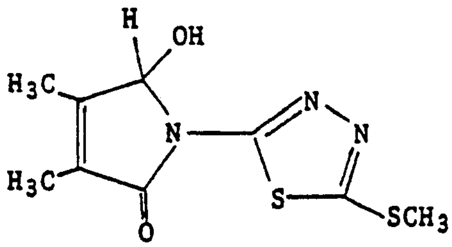

(The comparative compounds are shown in Japanese Patent Application Disclosure No. 23965-1978.)

Example 2

Submerged application test against lowland weeds under flooding condition

Formulation of Active Compounds

Carrier : 5 parts by weight of acetone
Emulsifier: 1 part by weight of benzyloxy polyglycol ether

To produce a suitable preparation of active compound, 1 part by weight of active compound was mixed with the stated amount of carrier and with the stated amount of emulsifier, and the resulting emulsifiable concentrate was then diluted with water to the desired concentrations.

Test Procedures

Each of several pots, having a size of 25 x 20 x 9 cm and an area of 1/2,000 are, was filled with soil taken out from a paddy field. Young paddy-rice plants (Nipponbare Variety) of the 2.5-leaf stage, with a height of 15 cm, were transplanted into these pots. Each pot had two zones, with the number of the plants grown in each zone being three. Then, seeds of the following weeds were sown in the soil, which was kept under wet conditions:
barnyard grass (Echinochloa crus-galli);
umbrella plant (Cyperus diformis);
monochoria (Monochoria vaginalis); and
annual broad-leaved weeds such as false pimpernel (Lindernia pyxidaria), toothcup (Rotala indica), American waterwort (Elatine triandra), red stem (Ammannia multiflora), and dopatrium (Dopatrium).

After 2 days, a large amount of water was supplied into each of the pots, so that a sheet of water with a thickness of 2 - 3 cm, was formed over the soil in each pot. Five days after the transplantation of the rice plants, the emulsion of the active compound, which had been prepared in the manner mentioned above, was applied to the pots in a predetermined amount by means of a pipette. After that, the water sheet was kept in a thickness of about 3 cm.

Four weeks after the application of the active compound, the degree of damage to the weeds and the degree of phytotoxicity on the paddy-rice plants were determined, and recorded according to the following

assessment scale.

| Rating | Herbicidal effect of active compound on weed in % * |
|---|---|
| 5 | 95% or more (fatal effect) |
| 4 | at least 80% and less than 95% |
| 3 | at least 50% and less than 80% |
| 2 | at lease 30% and less than 50% |
| 1 | at least 10% and less than 30% |
| 0 | less than 10% (no herbicidal effect) |
| **Rating** | **Phytotoxic effect of active compound on crops in % *** |
| 5 | at least 90% (fatal phytotoxicity) |
| 4 | at least 50% and less than 90% |
| 3 | at least 30% and less than 50% |
| 2 | at least 10% and less than 30% |
| 1 | more than 0% and less than 10% |
| 0 | 0% (no phytotoxicity) |

* These values (%) are those obtained by comparing the test data in the treated section with the test data in the control (untreated) section.

The test results are shown in Table 2.

Table 2

| Active compound No. | Amount of active compound (kg/ha) | Herbicidal effect on weeds | | | | Phytotoxic effect on rice plants |
|---|---|---|---|---|---|---|
| | | Barnyard grass | Umbrella plant | Monochoria | Annual broad-leaved grass | |
| 1 | 0.25 | 3 | 4 | 5 | 5 | 0 |
| 4 | 0.25 | 5 | 5 | 5 | 5 | 1 |
| 14 | 0.25 | 4 | 5 | 5 | 5 | 0 |
| 15 | 0.25 | 4 | 5 | 5 | 5 | 0 |
| (Known compound) | | | | | | |
| E-1 | 0.25 | 0 | 0 | 0 | 0 | 0 |
| E-2 | 0.25 | 0 | 0 | 0 | 0 | 0 |

Example 3

Pre-emergence soil treatment test against upland weeds

In a greenhouse, a number of pots, each having an area of 500 cm$^2$, were charged with soil obtained from a cultivated field. Seeds of maize plant were sown into the soil in the pots. Thereafter the surface of the soil was covered with a soil layer. The soil layer contained seeds of fingergrass (Digitaria), wild blite

(Amaranthus) and goosefoot (Chenopodium). The thickness of the soil layer was about 1 cm.

One day after the sowing, a predetermined amount of the preparation of the active compound, which had been produced in the same manner as in Example 2, was uniformly applied to the top soil layer in each pot

Four weeks after the application of the active compound, the degree of damage to the weeds and the degree of phytotoxicity on the crops were determined in the same manner as in Example 2. The test results are shown in Table 3.

Table 3

| Active compound No. | Amount of active compound (kg/ha) | Herbicidal effect on weeds | | | Phytotoxic effect on maize plants |
|---|---|---|---|---|---|
| | | Fingergrass | Wild blite | Goosefoot | |
| 2 | 0.5 | 4 | 5 | 5 | 0 |
| 4 | 0.5 | 5 | 5 | 5 | 0 |
| 15 | 0.5 | 4 | 5 | 5 | 0 |
| (Known compound) | | | | | |
| E-1 | 0.5 | 0 | 1 | 1 | 0 |
| E-2 | 0.5 | 0 | 1 | 1 | 0 |

Example 4

Herbicidal test by foliage application on upland weeds.

In a greenhouse, a number of pots, each having an area of 500 $cm^2$, were charged with soil obtained from a cultivated field. Seeds of maize plant (Indian corn) were sown onto the soil in the pots. Thereafter, the surface of the soil was covered with a soil layer. The soil layer contained seeds of barnyard grass (Echinocloa), fingergrass (Digitaria), wild blite (Amaranthus) and goosefoot (Chenopodium). The thickness of the soil layer was about 1 cm.

The maize plants were grown for 14 days, and then the plants were uniformly sprayed with a predetermined amount of the formulation of the active compound which had been produced in the same manner as in Example 2.

Four weeks after the application of the active compound, the degree of damage to the weeds and the degree of phytotoxicity on the maize plants were determined in the same manner as in Example 2. The test results are shown in Table 4.

Table 4

| Active compound No. | Amount of active compound (kg/ha) | Herbicidal effect on weeds | | | | Phytotoxicity on maize plants |
|---|---|---|---|---|---|---|
| | | Barnyard grass | Fingergrass | Wild blite | Goosefoot | |
| 1 | 0.25 | 4 | 5 | 5 | 5 | 0 |
| 2 | 0.25 | 3 | 4 | 5 | 5 | 0 |
| 4 | 0.25 | 4 | 5 | 5 | 5 | 0 |
| 14 | 0.25 | 3 | 5 | 4 | 5 | 1 |
| 15 | 0.25 | 4 | 5 | 5 | 5 | 0 |
| (Known compound) | | | | | | |
| E-1 | 0.25 | 0 | 0 | 1 | 1 | 0 |
| E-2 | 0.25 | 0 | 0 | 1 | 1 | 0 |

**Claims**

1. 3,4-Dimethyl-pyrrolidine derivatives of the formula (I)

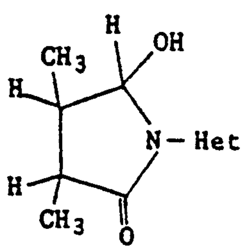

(I)

wherein Het represents a 5- or 6-membered heterocyclic radical containing 1 to 4 heteroatoms and optionally having a fused benzene ring, with the proviso that at least one of the heteroatoms is nitrogen, and that, when there are at least two heteroatoms, these heteroatoms may be identical or different from each other, and wherein Het may optionally be mono-, di-, tri- or tetra-substituted with identical or different radicals selected from the class consisting of halogen, $C_{1-8}$ alkyl, $C_{1-8}$ haloalkyl, unsubstituted or methyl-substituted $C_{1-6}$ cycloalkyl, $C_{1-4}$ alkoxy, $C_{1-4}$ haloalkoxy, $C_{1-4}$ alkylsulfonyl and $C_{1-4}$ haloalkylsulfonyl.

2. The compounds according to claim 1 wherein Het represents a 5- or 6-membered heterocyclic radical containing 1 to 3 heteroatoms and optionally having a fused benzene ring, with the proviso that at least one of the heteroatoms is nitrogen, and that, when there are at least two heteroatoms, these heteroatoms may be identical or different from each other, and wherein Het may optionally be mono-, di- or tri-substituted with identical or different radicals selected from the class consisting of fluorine, chlorine, $C_{1-6}$ alkyl, $C_{1-6}$ haloalkyl, 1-methyl-cyclopropan-1-yl, 1-methyl-cyclopentan-1-yl, 1-methyl-cyclohexan-1-yl, methoxy, ethoxy, trifluoromethoxy, 2,2,2-trifluoroethoxy, methylsulfonyl, ethylsulfonyl, n-propylsulfonyl, iso-propylsulfonyl, tert-butylsulfonyl, trifluoromethylsulfonyl and 2,2,2-trifluoroethylsulfonyl.

3. The compounds according to claim 1, wherein Het represents a 5- or 6-membered heterocyclic radical containing 1 to 3 heteroatoms and optionally having a fused benzene ring, with the proviso that at least one of the heteroatoms is nitrogen, and that, when there are at least two heteroatoms, these heteroatoms may be identical or different from each other, and wherein Het is optionally mono- or di-substituted with identical or different radicals selected from the class consisting of fluorine, chlorine, methyl, isopropyl, tert-butyl, 1-ethyl-1-methylpropyl, 1,1-dimethylpropyl, 2-chloro-1,1-dimethylethyl, 2-fluoro-1,1-dimethylethyl, trifluoromethyl, 1-methylcyclopropan-1-yl, trifluoromethoxy, methylsulfonyl, ethylsulfonyl, iso-propylsulfonyl, tert.-butylsulfonyl and trifluoromethylsulfonyl.

4. The compounds according to claim 1, wherein the heterocyclic radical is selected from the class consisting of pyrrole, furan, thiophene, pyrazole, imidazole, oxazole, isoxazole, thiazole, isothiazole, triazole, oxadiazole, thiadiazole, pyridine, pyrimidine, pyridazine, pyrazine, oxazine, thiazine, triazine, oxadiazine, thiadiazine, indole, iso-indole, benzofuran, benzothiophene, benzimidazole, indazole, benzoxazole, benzothiazole, quinoline, isoquinoline, cinnoline, quinazoline, quinoxaline, phthalazine, naphthyridine, benzoxazine and benzothiazine.

5. Process for the preparation of 3,4-dimethyl-pyrrolidine derivative of the formula (I)

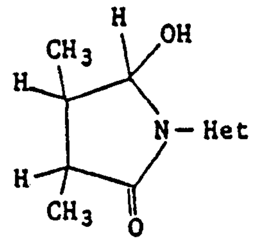

(I)

wherein Het represents a 5- or 6-membered heterocyclic radical containing 1 to 4 heteroatoms and optionally having a fused benzene ring, with the proviso that at least one of the heteroatoms is nitrogen, and that, when there are at least two heteroatoms, these heteroatoms may be identical or different from each other, and wherein Het may optionally be mono-, di-, tri- or tetra-substituted with identical or different radicals selected from the class consisting of halogen, $C_{1-8}$ alkyl, $C_{1-8}$ haloalkyl, unsubstituted or methyl-substituted $C_{3-6}$ cycloalkyl, $C_{1-4}$ alkoxy, $C_{1-4}$ haloalkoxy, $C_{1-4}$ alkylsulfonyl and $C_{1-4}$ haloalkylsulfonyl, characterized in that
3,4-dimethyl-2,5-dioxopyrrolidine derivatives of the formula (II)

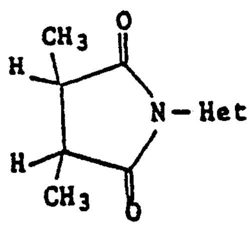

(II)

wherein Het has the meaning stated above,
are reacted with sodium borohydride, in the presence of inert solvents.

6. Herbicidal composition, characterized in that they contain at least one 3,4-dimethyl-pyrrolidine derivatives of the formula (I) according to claim 1.

7. Process for combating weeds, characterized in that 3,4-dimethyl-pyrrolidine derivatives of the formula (I) according to claim 1 are allowed to act on weeds and/or their habitat.

8. Use of 3,4-dimethyl-pyrrolidine derivatives of the formula (I) according to claim 1 for combating weeds.

9. Process for the preparation of herbicidal compositions, characterized in that 3,4-dimethyl-pyrrolidine derivatives of the formula (I) according to claim 1 are mixed with extenders and/or surface active agents.